# EUROPEAN PATENT APPLICATION

(11) **EP 0 630 649 A1**
(43) Date of publication of application: **28.12.1994**
(21) Application number: 94304174.9
(22) Date of filing: 09.06.1994
(51) Int. Cl.: A61K 31/57, A61K 31/195

(54) **Combination of an inhibitor of inducible nitric oxide synthase (iNOS) and an antiinflammatory agent, e.g. a corticosteroid**

(30) Priority: 14.06.1993 GB 9312204
(71) Applicant: ZENECA LIMITED, London W1Y 6LN (GB)
(72) Inventor: Teale, David Michael, Macclesfield, Cheshire SK10 4TG (GB)
(74) Representative: Tinsley, Rachel Maria

(57) **Abstract**

The invention relates to a pharmaceutical composition comprising a preferential inhibitor of inducible nitric oxide synthase over constitutive nitric oxide synthase and an anti-inflammatory agent for the therapeutic treatment of the pathological consequences of systemic inflammatory response syndrome, a process for its preparation and its use.

## Description

This invention relates to a composition for the treatment of the pathological consequences of septic shock and related conditions. In its broadest aspect it relates to a composition for the treatment of the pathological consequences of systemic inflammatory response syndrome (SIRS). The invention also relates to the use of the composition in the treatment of the pathological consequences of SIRS and the use of the composition in the manufacture of a medicament for treatment of the pathological consequences of with SIRS. More specifically the invention relates to a composition comprising a preferential inhibitor of inducible nitric oxide synthase (iNOS) over constitutive nitric oxide synthase (cNOS), hereinafter known as preferential inhibitor of iNOS, and an anti-inflammatory agent or to the simultaneous, separate or sequential administration of the preferential inhibitor of iNOS and the anti-inflammatory agent as a treatment of the pathological consequences of SIRS. An example of a pathological consequence of SIRS is hypotension.

Insults which can result in SIRS include, trauma, burns, allergic reaction, hypovolaemia, cytokine therapy, blunt trauma, pancreatitis and infection. When SIRS is the result of an infection, it is termed sepsis. The physiological changes associated with SIRS include a body temperature greater than 38°C, or less than 35.5°C, an increased heart rate, tachypnea and an alteration in white blood cell count. Animals with SIRS frequently become hypotensive. For example, sepsis frequently leads to severe sepsis which is, as defined by R Bone (Definitions for sepsis and organ failure and guidelines for the use of innovative therapies in sepsis. Chest 1992; 101, 1644-1655), sepsis associated with organ dysfunction, hypoperfusion abnormality or sepsis-induced hypotension.

It has been established that over-production of nitric oxide is caused by iNOS, while cNOS is thought to play a role in normal blood pressure regulation. Over-production of nitric oxide is believed to contribute to the pathology of SIRS, e.g. hypotension associated with SIRS.

Tests to identify preferential inhibitors of iNOS are described in international patent application WO 92/16666.

Anti-inflammatory agents such as corticosteroids were once thought to be beneficial in the treatment of septic shock, an example of hypotension associated with SIRS.

Pre-infection treatment with dexamethasone has been shown to prevent subsequent pathology, for example hypotension, when an animal is subsequently exposed to LPS (lipopolysaccharide). However, the view that post-infection treatment with a corticosteroid is beneficial in the treatment of septic shock has changed over the years and current opinion is that treatment of patients with severe sepsis and septic shock should not include corticosteroid treatment, because it does not reduce mortality.

In the early 1980's the Food and Drug Administration (USA) reviewed the indications for corticosteroids in septic shock and decided to remove septic shock from the insert of Solu-Medrol (methylprednisolone sodium succinate) as an indication, because firm evidence of the benefit in septic shock was deemed not to exist.

In 1984, there was controversy as to whether it was beneficial to use corticosteroids in septic shock. A trial conducted by C L Sprung et al, reported in The New England Journal of Medicine 1984, vol. 311, No. 18, 1137, compared the effect of a two dose regimen of corticosteroid with that of a placebo in 59 patients with septic shock, but found no difference in mortality in the three groups.

In The New England Journal of Medicine 1987, vol. 317, No. 11, 653-658, R C Bone et al stated "In summary, our prospective, randomised, placebo-controlled, double blind multicenter trial in patients with severe sepsis and septic shock has shown no benefits from the use of high doses of corticosteroids, in contrast to previous reports showing substantial benefits from early administration. Our patients with sepsis or septic shock were identified early and were treated within two hours of diagnosis. No improvement was found in shock prevention, shock reversal, or mortality in 14 days. In addition, the patients who had renal insufficiency at entry and received methylprednisolone had a decreased rate of shock reversal and a significantly increased mortality. Although the incidence of secondary infection did not differ between the two treatment groups, the mortality attributed to secondary infections was significantly increased in the steroid group. These results strongly argue against the use of high-dose corticosteroids as an adjunct in severe sepsis and septic shock."

The Veterans Administration Systemic Sepsis Cooperative Study Group also conducted a multicentre randomised double blind placebo-controlled trial and concluded that "the treatment of patients with systemic sepsis, whether early or late (septic shock) should not include glucocorticoid therapy, because it does not improve survival." (The New England Journal of Medicine 1987, vol. 317, No. 11, 659-663).

We have now discovered that there is an increase in survival rate when animals inoculated intra-veinously with a lethal dose of LPS are treated therapeutically (post-insult) with a combination of a preferential inhibitor of iNOS and an anti-inflammatory agent compared with therapeutic treatment with a preferential inhibitor of iNOS without an anti-inflammatory agent.

Accordingly, the present invention provides a pharmaceutical composition comprising a preferential inhibitor of iNOS and an anti-inflammatory agent.

Suitable preferential inhibitors of iNOS include L-canavanine, aminoguanidine and N⁶-(hydrazinoiminomethyl)lysine.

Examples of anti-inflammatory agents include corticosteroids such as methylprednisolone, dexamethasone, betamethasone, prednisolone, hydrocortisone, triamicinolone, meprednisone and paramethasone; NSAIDs such as aspirin, aminopyrine, azapropazone, benoxaprofen, fenoprofen, ibuprofen, indomethacin and naproxen; inhibitors of the enzyme 5-lipoxygenase (such as those described in European patent applications Nos. 0351194, 0375368, 0375404, 0375452, 037547, 0381375, 0385662, 0385663, 0385679, 0385680); 5-lipoxygenase activating protein antagonists, leukotriene antagonists and bradykinin antagonists.

In a preferred aspect, the invention provides a pharmaceutical composition comprising a preferential inhibitor of iNOS and a corticosteroid.

In a more preferred aspect, the invention provides a pharmaceutical composition comprising a preferential inhibitor of iNOS and dexamethasone or methylprednisolone.

In another aspect, the invention provides a pharmaceutical composition comprising an anti-inflammatory agent and L-canavanine, aminoguanidine or N⁶-(hydrazinoiminomethyl)lysine. More preferably the pharmaceutical composition comprises dexamethasone or methylprednisolone and L-canavanine, aminoarginine or N⁶-(hydrazinoiminomethyl)lysine.

Most preferably the pharmaceutical composition comprises dexamethasone and L-canavanine.

Pharmaceutical compositions of the present invention are prepared by mixing an effective amount of the active ingredients with a pharmaceutically-acceptable carrier. For example, in parenteral compositions, the carrier may be sterile water. Preferably the composition is in a form suitable for parental administration including intravenous, subcutaneous, intramuscular, intravascular or infusion. The compositions may be prepared in a conventional manner using conventional excipients.

A specific example of a composition which is constituted as a 1% solution in water, freeze dried and may be made up by adding 0.9% aqueous sodium chloride solution to give the required concentration, is as follows:

| | |
|---|---|
| dexamethasone | 148mg |
| L-canavanine | 740mg |

It will be appreciated that a preferential inhibitor of iNOS and an anti-inflammatory agent may be brought into admixture when a pharmaceutical composition is prepared in a conventional manner. Alternatively, separate pharmaceutical compositions may be prepared, where one contains a preferential inhibitor of iNOS and the other an anti-inflammatory agent.

Accordingly, a further aspect of the invention provides a process for the preparation of a pharmaceutical composition for the therapeutic treatment of the pathological consequences of SIRS characterised by bringing into conjunction or admixture a preferential inhibitor of iNOS, an anti-inflammatory agent and a pharmaceutically-acceptable carrier.

The dosage of an anti-inflammatory agent administered to an animal suffering from hypotension associated with SIRS will normally be in the range of 0.3 mg/kg⁻¹ to 30 mg/kg⁻¹ and the dosage of preferential inhibitor of iNOS will normally be in the range of 0.1 mg/kg⁻¹ to 200 mg/kg⁻¹. Preferably the dosage of an anti-inflammatory agent is 1-10 mg/kg⁻¹. The size of the dose will naturally vary according to the nature of the preferential inhibitor of iNOS and anti-inflammatory agent, the nature and severity of the conditions and the route of administration, according to well known principles of medicine.

Clearly, the effective ingredients can be administered sequentially or separately as well as simultaneously. Accordingly, the invention also provides products containing a preferential inhibitor of iNOS and an anti-inflammatory agent as a combined preparation for simultaneous, separate or sequential use in the therapeutic treatment of the pathological consequences of SIRS. Those skilled in the art can select the appropriate dosage of each active ingredient for the therapeutic treatment of hypotension associated with SIRS.

In a preferred aspect, the invention relates to the therapeutic treatment of the pathological consequences of severe sepsis and septic shock.

A further aspect of the invention is the use of a preferential inhibitor of iNOS and an anti-inflammatory agent in the therapeutic treatment of the pathological consequences of SIRS.

A preferred aspect of the invention is the use of a preferential inhibitor of iNOS and a corticosteroid in the therapeutic treatment of the pathological consequences of severe sepsis and septic shock.

The invention also provides the use of a preferential inhibitor of iNOS and methylprednisolone or dexamethasone in the therapeutic treatment of SIRS and its associated pathology.

In another aspect the invention provides the use of L-canavanine, aminoguanidine or N⁶-(hydrazinoiminomethyl)lysine and a corticosteroid in the therapeutic treatment of the pathological consequences of SIRS.

Preferably, the preferential inhibitor of iNOS is L-canavanine, aminoguanidine or N⁶-(hydrazinoiminomethyl)lysine and the anti-inflammatory agent is methylprednisolone or dexamethasone.

Most preferably, the preferential inhibitor of iNOS is L-canavanine and the anti-inflammatory agent is dexamethasone.

The invention also provides the use of a preferential inhibitor of iNOS and an anti-inflammatory agent in the manufacture of a medicament for the therapeutic treatment of the pathological consequences of SIRS.

The invention also concerns a method of treatment of the pathological consequences of SIRS comprising administering an effective amount of a preferential inhibitor of iNOS and an effective amount of an anti-inflammatory agent to a patient in need thereof.

Preferably the invention relates to the treatment of hypotension associated with SIRS.

Most preferably the invention relates to the treatment of hypotension associated with severe sepsis and septic shock.

The invention will now be illustrated by the following example which illustrate the advantage in the therapeutic treatment of SIRS with a combination of a preferential inhibitor of iNOS and an anti-inflammatory agent.

### Example

The in vivo efficacy of dexamethasone, L-canavanine or a combination of the two was tested in a rat model of endotoxaemia. Male Alderley Park rats (120-130 grams) were challenged intravenously with a previously determined LD90 of LPS (lipopolysaccharide) (Sigma E. coli 0111:34) 60 mg/kg) at time zero, and subsequently dosed intravenously 3-3.5 hours later with either dexamethasone (3 mg/kg), L-canavanine (30 mg/kg or 100 mg/kg) or a combination of the two (3 mg dexamethasone/kg + 30 mg L-canavanine/kg or 3 mg dexamethasone + 100 mg L-canavanine/kg). Control animals received either saline intravenously 3-4 hours after LPS administration or 3 mg dexamethasone/kg 90 minutes before LPS administration. Survival was monitored over a 72 hour observation period.

11% of saline dosed control animals and 100% of dexamethasone pretreated animals survived the 72 hour observation period. In those animals receiving 3 mg dexamethasone/kg, 30 mg L-canavanine/kg or 100 mg L-canavanine/kg, 3-3.5 hours after LPS challenge, survival was 43% (27/62), 17% (4/24) and 5% (1/21) respectively. In contrast, those animals receiving a combination of dexamethasone and L-canavanine, 3-3.5 hours following LPS challenge, had a significant improvement in survival, with 71% (17/24) surviving in the 3 mg dexamethasone + 30 mg L-canavanine/kg group (P< 0.05 compared to dexamethasone alone and P<0.001 compared to L-canavanine alone), and 95% (19/20) surviving in the 3 mg dexamethasone/kg + 100 mg L-canavanine/kg group (P<.001 compared with either dexamethsone or L-canavanine alone).

| Dosing regimen | Survival |
|---|---|
| Control | 11% (6/54) |
| Dexamethasone (-90 minutes) (3mg/kg) | 100% (60/60) |
| Dexamethasone (3 mg/kg) | 43% (27/62) |
| L-canavanine (30 mg/kg) | 17% (4/24) |
| L-canavanine (100 mg/kg) | 5% (1/21) |
| Dexamethasone + L-canavanine (3 mg/kg+30 mg/kg) | 71% (17/24) |
| Dexamethasone + L-canavanine (3 mg/kg+100 mg/kg) | 95% (19/20) |

## Claims

1. A pharmaceutical composition comprising a preferential inhibitor of iNOS and an anti-inflammatory agent.

2. A pharmaceutical composition as defined in claim 1 wherein the anti-inflammatory agent is a corticosteroid.

3. A pharmaceutical composition as defined in claim 1 wherein the anti-inflammatory agent is dexamethasone or methylprednisolone.

4. A pharmaceutical composition as defined in any one of claims 1 to 3 wherein the preferential inhibitor of iNOS is L-canavanine, aminoguanidine or N⁶-(hydrazinoiminomethyl)lysine.

5. A pharmaceutical composition as defined in any one of claims 1 to 3 wherein the preferential inhibitor of iNOS is L-canavanine.

6. A product containing a preferential inhibitor of iNOS and an anti-inflammatory agent as a combined preparation for simultaneous, or separate or sequential use in the therapeutic treatement of the pathological consequences of SIRS.

7. A process for preparing a pharmaceutical composition as defined in any one of claims 1 to 5 comprising mixing a preferential inhibitor of iNOS and an anti-inflammatory agent with a pharmaceutically-acceptable carrier.

8. A pharmaceutical composition as defined in any one of claims 1 to 5 for use in the therapeutic treatment of the pathological consequences of SIRS.

9. A pharmaceutical composition as defined in any one of claims 1 to 5 for use as a medicament.

10. A pharmaceutical composition as defined in any one of claims 1 to 5 for use in the therapeutic treatment of hypotension associated with SIRS.

11. The use of a preferential inhibititor of iNOS and an anti-inflammatory agent for the manufacture of a medicament for the therapeutic treatment of the pathological consequences of SIRS.
